# EUROPEAN PATENT APPLICATION

(11) **EP 0 524 834 A2**
(43) Date of publication of application: **27.01.1993**
(21) Application number: 92306803.5
(22) Date of filing: 24.07.1992
(51) Int. Cl.: C12N 15/57, C12N 9/64, A61K 37/54, C12N 1/21

(54) **Immunosuppressive drugs containing a cysteine protease**

(30) Priority: 25.07.1991 JP 208546/91; 12.02.1992 JP 57189/92
(71) Applicant: Hamajima, Fusanori, Nerima-ku, Tokyo (JP); Yamamoto, Mikio, Tokorazawa-shi, Saitama-ken (JP); Tsuru, Sumiaki, Tokyo (JP); Yamakami, Kazuo, Wako-shi, Saitama-ken (JP)
(72) Inventor: Hamajima, Fusanori, Nerima-ku, Tokyo (JP); Yamamoto, Mikio, Tokorazawa-shi, Saitama-ken (JP); Tsuru, Sumiaki, Tokyo (JP); Yamakami, Kazuo, Wako-shi, Saitama-ken (JP)
(74) Representative: Cresswell, Thomas Anthony

(57) **Abstract**

The invention provides a cysteine protease for use in treatment of the human or animal body by therapy. The protease is capable of suppressing the human or animal immune system, and inhibits delayed-type immune hypersensitivity and antibody production against specific antigens and graft tissue. The protease is obtainable form parasitic helminths.

SEQUENCE LISTING
(1) GENERAL INFORMATION:
(i) APPLICANT:
(ii) TITLE OF INVENTION:
(iii) NUMBER OF SEQUENCES:
(iv) CORRESPONDENCE ADDRESS:
(A) ADDRESSEE:
(B) STREET:
(C) CITY:
(D) STATE:
(E) COUNTRY:
(F) ZIP:
(v) COMPUTER READABLE FORM;
(A) MEDIUM TYPE:
(B) COMPUTER:
(C) OPERATING SYSTEM:
(D) SOFTWARE:
(vii) ATTORNEY/AGENT INFORMATION:
(A) NAME:
(B) REGISTRATION NUMBER:
(C) REFERENCE/DOCKET NUMBER:
(ix) TELECOMMUNICATION INFORMATION:
(A) TELEPHONE:
(B) TELEFAX:

(2) INFORMATION FOR SEQ ID NO.1
(i) SEQUENCE CHARACTERISTICS:
(A) LENGTH:
(B) TYPE: amino acid
(C) STRANDEDNESS: -
(D) TOPOLOGY: Linear
(ii) MOLECULE TYPE: peptide
(vi) ORIGINAL SOURCE
(A) ORGANISM: Paragonimus westermani metacecaria
(B) CELL TYPE:
(C) CELL LINE:
(ix) FEATURES

(ix)-(1)
(A) LOCATION: 15
(B) NAME/KEY: Xaa=Ala or Pro

(ix)-(2)
(A) LOCATION: 21
(B) NAME/KEY: Xaa=Ser or Glu

(ix)-(3)
(A) LOCATION: 58
(B) NAME/KEY: Xaa=Arg or Met

(ix)-(4)
(A) LOCATION: 59
(B) NAME/KEY: Xaa=Val or Ala

(ix)-(5)
(A) LOCATION: 61
(B) NAME/KEY: Xaa=Gln or Glu

(ix)-(6)
(A) LOCATION: 69
(B) NAME/KEY: Xaa=Ala or Ser

(ix)-(7)
(A) LOCATION: 77
(B) NAME/KEY: Xaa=Tyr or Asp

(xi) SEQUENCE DESCRIPTION: SEQ ID NO.1

1 10 20
Ala Pro Glu Arg Ile Asp Trp Arg Ala Lys Gly Ala Val Thr Xaa Val Glu Asn Gln Gly
21 30 40
Xaa Cys Gly Ser Cys Trp Ala Phe Ser Thr Ala Gly Asn Val Glu Gly Gln Trp Phe Ile
41 50 60
Lys Thr Gly Gln Leu Val Ser Leu Ser Lys Gln Gln Leu Val Asp Cys Asp Xaa Xaa Ala
61 70 80
Xaa Gly Cys Asn Gly Gly Trp Pro Xaa Ser Ser Tyr Leu Glu Ile Met Xaa Met Gly Gly
81 90 100
Leu Glu Ser Glu Ser Asp Tyr Pro Tyr Val Gly Val Glu Gln Thr Cys Ala Leu Asn Lys
101 110 120
Glu Lys Leu Val Ala Lys Ile Asp Asp Ser Ile Val Leu Gly Pro Glu Glu Glu Asp His
121 130 140
Ala Ala Tyr Leu Ala Glu His Gly Pro Leu Ser Thr Leu Leu Asn Ala Val Ala Leu Gln

141 150 160

Tyr Tyr Gln Ser Gly Val Leu Lys Pro Thr Phe Glu Glu Cys Pro Asp Thr Glu Leu Asn

161 170 180

His Ala Val Leu Thr Val Gly Tyr Asp Lys Glu Gly Asp Met Pro Tyr Trp Ile Ile Lys

181 190 200

Asn Ser Trp Gly Thr Asp Trp Gly Glu Lys Gly Tyr Phe Arg Leu Phe Arg Gly Asp Cys

201 210

Thr Cys Gly Ile Asn Arg Met Ala Thr Ser Ala Ile Ile Lys Lys ***

(2) INFORMATION FOR SEQ ID NO.2
(i) SEQUENCE CHARACTERISTICS:
(A) LENGTH:
(B) TYPE: nucleic acid
(C) STRANDEDNESS:
(D) TOPOLOGY:
(ii) MOLECULE TYPE: cDNA to mRNA
(vi) ORIGINAL SOURCE
(A) ORGANISM: Paragonimus westermani metacecaria
(B) CELL TYPE:
(C) CELL LINE:
(ix) FEATURES

(ix)-(1)
(A) LOCATION: 15
(B) NAME/KEY: N=T or G

(ix)-(2)
(A) LOCATION: 43
(B) NAME/KEY: N=G or C

(ix)-(3)
(A) LOCATION: 48
(B) NAME/KEY: N=T or G

(ix)-(4)
(A) LOCATION: 61
(B) NAME/KEY: N=T or G

(ix)-(5)
(A) LOCATION: 62
(B) NAME/KEY: N=C or A

(ix)-(6)
(A) LOCATION: 66
(B) NAME/KEY: N=C or T

(ix)-(7)
(A) LOCATION: 123
(B) NAME/KEY: N=A or G

(ix)-(8)
(A) LOCATION: 173
(B) NAME/KEY: N=G or T

(ix)-(9)
(A) LOCATION: 176
(B) NAME/KEY: N=T or C

(ix)-(10)
(A) LOCATION: 181
(B) NAME/KEY: N=C or G

(ix)-(11)
(A) LOCATION: 205
(B) NAME/KEY: N=G or T

(ix)-(12)
(A) LOCATION: 213
(B) NAME/KEY: N=C or A

(ix)-(13)
(A) LOCATION: 229
(B) NAME/KEY: N=T or G

(ix)-(14)
(A) LOCATION: 237
(B) NAME/KEY: N=C or T

(ix)-(15)
(A) LOCATION: 306
(B) NAME/KEY: N=G or A

(ix)-(16)
(A) LOCATION: 366
(B) NAME/KEY: N=C or T

(ix)-(17)
(A) LOCATION: 370
(B) NAME/KEY: N=T or C

(ix)-(18)
(A) LOCATION: 375
(B) NAME/KEY: N=A or T

(ix)-(19)
(A) LOCATION: 471
(B) NAME/KEY: N=T or C

(ix)-(20)
(A) LOCATION: 489
(B) NAME/KEY: N=T or G

(A) OTHER INFORMATION:

(xi) SEQUENCE DESCRIPTION: SEQ ID NO.2

1 60
GCT CCC GAA CGT ATN GAC TGG CGG GCT AAG GGC GCT GTG ACA NCG GTN GAA AAT CAA GGC

61 120
NNG TGN GGT TCG TGT TGG GCG TTC TCG ACT GCC GGA AAC GTT GAA GGT CAA TGG TTC ATC

121 180
AAN ACC GGT CAG CTT GTC AGT CTG AGC AAA CAG CAA TTG GTC GAC TGT GAC ANG GNG GCC

181 240
NAG GGA TGC AAT GGT GGA TGG CCA NCC AGT TCN TAC CTG GAA ATC ATG NAT ATG GGN GGT

241 300
TTG GAG TCC GAA AGC GAC TAT CCC TAT GTT GGT GTG GAA CAA ACG TGT GCC CTG AAC AAG

301 360
GAG AAN CTG GTA GCC AAA ATC GAT GAT TCG ATT GTT CTG GGT CCG GAG GAG GAG GAC CAC

361 420
GCC GCN TAT NTG GCN GAA CAC GGA CCG TTG AGT ACG CTG CTC AAT GCC GTC GCT CTT CAG

421 480
TAC TAC CAG TCC GGA GTA CTC AAA CCG ACC TTT GAG GAG TGT CCG GAT ACN GAG TTG AAC

481 540
CAC GCG GTN CTC ACC GTC GGC TAT GAC AAG GAA GGC GAT ATG CCA TAC TGG ATC ATC AAG

541 600
AAT AGT TGG GGT ACC GAC TGG GGC GAG AAA GGC TAC TTC CGA CTC TTC CGA GGA GAT TGC

601
ACG TGT GGA ATC AAC CGC ATG GCA ACA TCC GCG ATC ATC AAG AAA TGA

(3) INFORMATION FOR SEQ ID NO.3
(i) SEQUENCE CHARACTERISTICS:
(A) LENGTH:
(B) TYPE: nucleic acid
(C) STRANDEDNESS:
(D) TOPOLOGY:
(ii) MOLECULE TYPE: cDNA to mRNA
(vi) ORIGINAL SOURCE
(A) ORGANISM: Paragonimus westermani metacecaria
(B) CELL TYPE:
(C) CELL LINE:
(ix) FEATURES
(A) OTHER INFORMATION:

(xi) SEQUENCE DESCRIPTION: SEQ ID NO.3

1 60
GCT CCC GAA CGT ATT GAC TGG CGG GCT AAG GGC GCT GTG ACA GCG GTT GAA AAT CAA GGC
Ala Pro Glu Arg Ile Asp Trp Arg Ala Lys Gly Ala Val Thr Ala Val Glu Asn Gln Gly

61 120
TCG TGC GGT TCG TGT TGG GCG TTC TCG ACT GCC GGA AAC GTT GAA GGT CAA TGG TTC ATC
Ser Cys Gly Ser Cys Trp Ala Phe Ser Thr Ala Gly Asn Val Glu Gly Gln Trp Phe Ile

121 180
AAA ACC GGT CAG CTT GTC AGT CTG AGC AAA CAG CAA TTG GTC GAC TGT GAC AGG GTG GCC
Lys Thr Gly Gln Leu Val Ser Leu Ser Lys Gln Gln Leu Val Asp Cys Asp Arg Val Ala

181 240
CAG GGA TGC AAT GGT GGA TGG CCA GCC AGT TCC TAC CTG GAA ATC ATG TAT ATG GGC GGT
Gln Gly Cys Asn Gly Gly Trp Pro Ala Ser Ser Tyr Leu Glu Ile Met Tyr Met Gly Gly

241 300
TTG GAG TCC GAA AGC GAC TAT CCC TAT GTT GGT GTG GAA CAA ACG TGT GCC CTG AAC AAG
Leu Glu Ser Glu Ser Asp Tyr Pro Tyr Val Gly Val Glu Gln Thr Cys Ala Leu Asn Lys

301 360
GAG AAG CTG GTA GCC AAA ATC GAT GAT TCG ATT GTT CTG GGT CCG GAG GAG GAG GAC CAC
Glu Lys Leu Val Ala Lys Ile Asp Asp Ser Ile Val Leu Gly Pro Glu Glu Glu Asp His

361 420
GCC GCC TAT TTG GCA GAA CAC GGA CCG TTG AGT ACG CTG CTC AAT GCC GTC GCT CTT CAG
Ala Ala Tyr Leu Ala Glu His Gly Pro Leu Ser Thr Leu Leu Asn Ala Val Ala Leu Gln

421 480
TAC TAC CAG TCC GGA GTA CTC AAA CCG ACC TTT GAG GAG TGT CCG GAT ACT GAG TTG AAC
Tyr Tyr Gln Ser Gly Val Leu Lys Pro Thr Phe Glu Glu Cys Pro Asp Thr Glu Leu Asn

481 540
CAC GCG GTT CTC ACC GTC GGC TAT GAC AAG GAA GGC GAT ATG CCA TAC TGG ATC ATC AAG
His Ala Val Leu Thr Val Gly Tyr Asp Lys Glu Gly Asp Met Pro Tyr Trp Ile Ile Lys

541 600
AAT AGT TGG GGT ACC GAC TGG GGC GAG AAA GGC TAC TTC CGA CTC TTC CGA GGA GAT TGC
Asn Ser Trp Gly Thr Asp Trp Gly Glu Lys Gly Tyr Phe Arg Leu Phe Arg Gly Asp Cys

601
ACG TGT GGA ATC AAC CGC ATG GCA ACA TCC GCG ATC ATC AAG AAA TGA
Thr Cys Gly Ile Asn Arg Met Ala Thr Ser Ala Ile Ile Lys Lys •••

(4) INFORMATION FOR SEQ ID NO.4
(i) SEQUENCE CHARACTERISTICS:
(A) LENGTH:
(B) TYPE: nucleic acid
(C) STRANDEDNESS:
(D) TOPOLOGY:
(ii) MOLECULE TYPE: cDNA to mRNA
(vi) ORIGINAL SOURCE
(A) ORGANISM: Paragonimus westermani metacecaria
(B) CELL TYPE:
(C) CELL LINE:
(ix) FEATURES
(A) OTHER INFORMATION:

(xi) SEQUENCE DESCRIPTION: SEQ ID NO.4

1 60
GCT CCC GAA CGT ATG GAC TGG CGG GCT AAG GGC GCT GTG ACA CCG GTG GAA AAT CAA GGC
Ala Pro Glu Arg Ile Asp Trp Arg Ala Lys Gly Ala Val Thr Pro Val Glu Asn Gln Gly

61 120
GAG TGT GGT TCG TGT TGG GCG TTC TCG ACT GCC GGA AAC GTT GAA GGT CAA TGG TTC ATC
Glu Cys Gly Ser Cys Trp Ala Phe Ser Thr Ala Gly Asn Val Glu Gly Gln Trp Phe Ile

121 180
AAG ACC GGT CAG CTT GTC AGT CTG AGC AAA CAG CAA TTG GTC GAC TGT GAC ATG GCG GCC
Lys Thr Gly Gln Leu Val Ser Leu Ser Lys Gln Gln Leu Val Asp Cys Asp Met Ala Ala

181 240
GAG GGA TGC AAT GGT GGA TGG CCA TCC AGT TCA TAC CTG GAA ATC ATG GAT ATG GGT GGT
Glu Gly Cys Asn Gly Gly Trp Pro Ser Ser Ser Tyr Leu Glu Ile Met Asp Met Gly Gly

241 300
TTG GAG TCC GAA AGC GAC TAT CCC TAT GTT GGT GTG GAA CAA ACG TGT GCC CTG AAC AAG
Leu Glu Ser Glu Ser Asp Tyr Pro Tyr Val Gly Val Glu Gln Thr Cys Ala Leu Asn Lys

301 360
GAG AAA CTG GTA GCC AAA ATC GAT GAT TCG ATT GTT CTG GGT CCG GAG GAG GAG GAC CAC
Glu Lys Leu Val Ala Lys Ile Asp Asp Ser Ile Val Leu Gly Pro Glu Glu Glu Asp His

361 420
GCC GCT TAT CTG GCT GAA CAC GGA CCG TTG AGT ACG CTG CTC AAT GCC GTC GCT CTT CAG
Ala Ala Tyr Leu Ala Glu His Gly Pro Leu Ser Thr Leu Leu Asn Ala Val Ala Leu Gln

421 480
TAC TAC CAG TCC GGA GTA CTC AAA CCG ACC TTT GAG GAG TGT CCG GAT ACC GAG TTG AAC
Tyr Tyr Gln Ser Gly Val Leu Lys Pro Thr Phe Glu Glu Cys Pro Asp Thr Glu Leu Asn

481 540
CAC GCG GTG CTC ACC GTC GGC TAT GAC AAG GAA GGC GAT ATG CCA TAC TGG ATC ATC AAG
His Ala Val Leu Thr Val Gly Tyr Asp Lys Glu Gly Asp Met Pro Tyr Trp Ile Ile Lys

541 600
AAT AGT TGG GGT ACC GAC TGG GGC GAG AAA GGC TAC TTC CGA CTC TTC CGA GGA GAT TGC
Asn Ser Trp Gly Thr Asp Trp Gly Glu Lys Gly Tyr Phe Arg Leu Phe Arg Gly Asp Cys

601
ACG TGT GGA ATC AAC CGC ATG GCA ACA TCC GCG ATC ATC AAG AAA TGA
Thr Cys Gly Ile Asn Arg Met Ala Thr Ser Ala Ile Ile Lys Lys ***

(5) INFORMATION FOR SEQ ID NO.5
(i) SEQUENCE CHARACTERISTICS:
(A) LENGTH:
(B) TYPE: amino acid
(C) STRANDEDNESS:
(D) TOPOLOGY:
(ii) MOLECULE TYPE: peptide
(vi) ORIGINAL SOURCE
(A) ORGANISM: Paragonimus westermani metacecaria
(B) CELL TYPE:
(C) CELL LINE:
(ix) FEATURES
(A) OTHER INFORMATION:
The 22nd and 25th residues, Xaa, are to be cysteine which could not be
detected with this PTH analyser system. The amino acid sequence shown
below is N-terminal 25 amino acids of the purified native cysteine
protease.

(xi) SEQUENCE DESCRIPTION: SEQ ID NO.5

1 5 10 15
Ala Pro Glu Ser Ile Asp Trp Arg Glu Lys Gly Ala Val Ala Pro

16 20 25
Val Glu Asp Gln Gly Ser Xaa Gly Ser Xaa

## Description

### Field of the Invention

The present invention relates to immunosuppressive drugs used for suppression of graft rejection in organ transplantation or suppression of autoimmune diseases.

### Background of the Invention

Currently, azathiopline, cyclosporin A, FK-506, and 15-deoxysperguarine are known as immunosuppressive drugs used for a therapeutic purpose, and have potent suppression effect.

However, any of the conventional immunosuppressive drug do not induce immunological tolerance and are therefore required that the drug be continuously administered for a long period of time. As the result of long continuous administration, there have been the problem of side effects such as cancer, nephrotoxicity, angiitis, hepatotoxicity and disorders in the digestive tract.

The present inventors have studied on agents of human parasites, especially,parasitic helminths, which protects them from the immune response of human hosts, and found that cysteine protease, a secretory protein accumulated in the tissue of parasitic helminths, suppresses the cell-mediated and humoral immunities of mammalian hosts and induces the hosts to acquire immunological tolerance to the parasite.

### Summary of the Invention

The immunosuppressive drug of the present invention can suppress both delayed-type hypersensitivity and antibody production against specific antigens and graft tissues, and induce immunological tolerance by several administrations. Conventional immunosuppressive drugs are required that they be administered for a long period of tine, and cause an adversary effect which is a drawback of the drugs. In contrast, the immunosuppressive drug of the present invention is not required that it be administered for a long period of time. Therefore, the immunosuppressive drug of the invention overcomes the drawback of the conventional immunosuppressive drugs.

### Description of the Figures

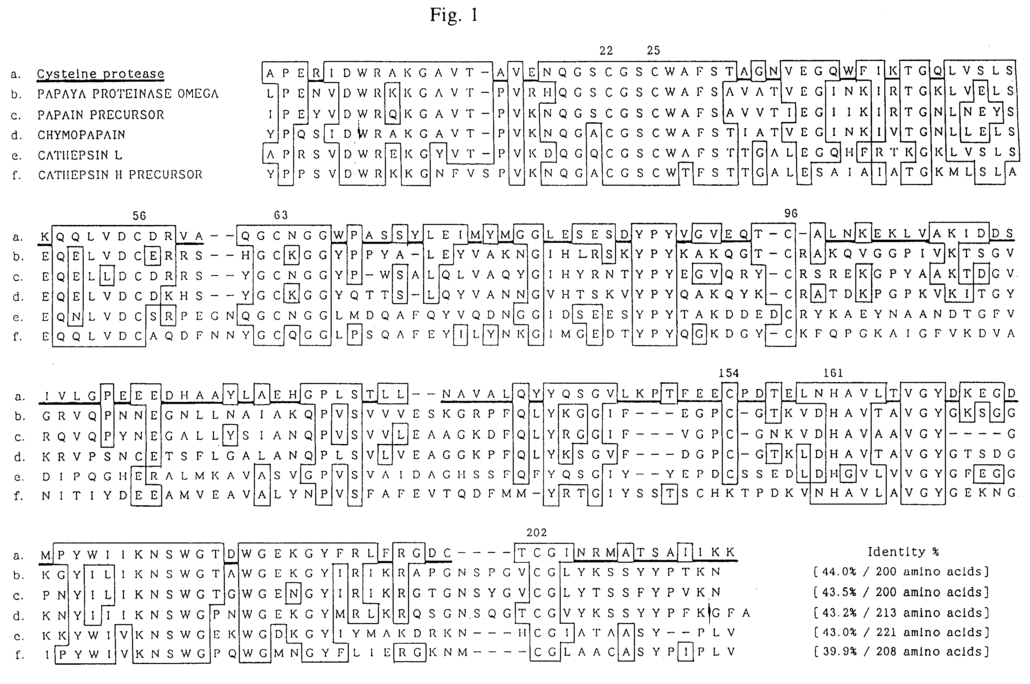

Fig. 1 shows a comparison of the deduced amino acid sequences of a cysteine protease from *Paragonimus westermani* metacercariae and other similar cysteine proteases. Homologous areas are boxed and heterologous amino acids are underlined.

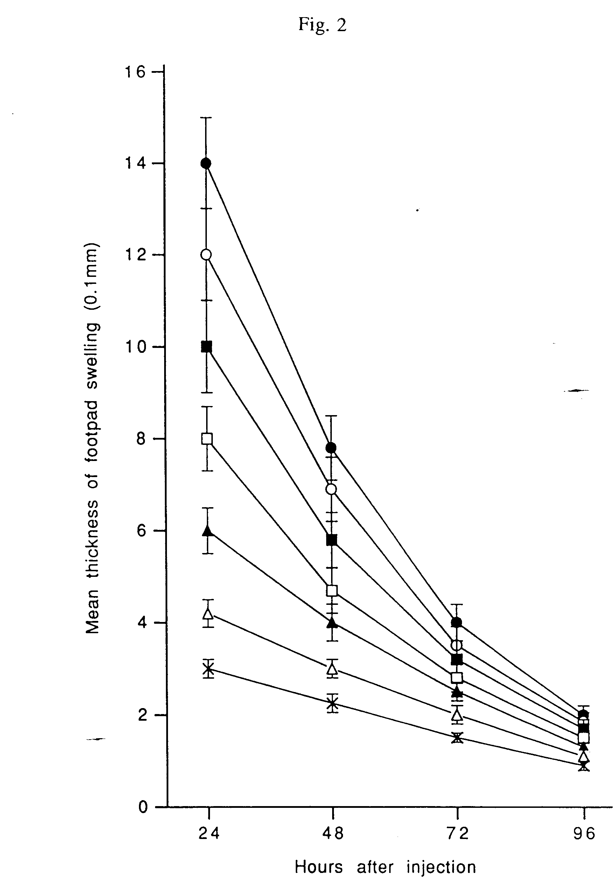

Fig. 2 is a graph showing a footpad response (swelling of footpad) of C₅₇BL/6 female mice. The female mice were intraperitoneally injected with sheep red blood cells (SRBC) at various times after intraperitoneal injection of the present cysteine protease. The intervals were 0 (X), 1 (Δ), 2 (▲), 3 and 4(□), 5 and 6(■), 7 and 8 days (○) and untreated control (●). Bars represent standard errors.

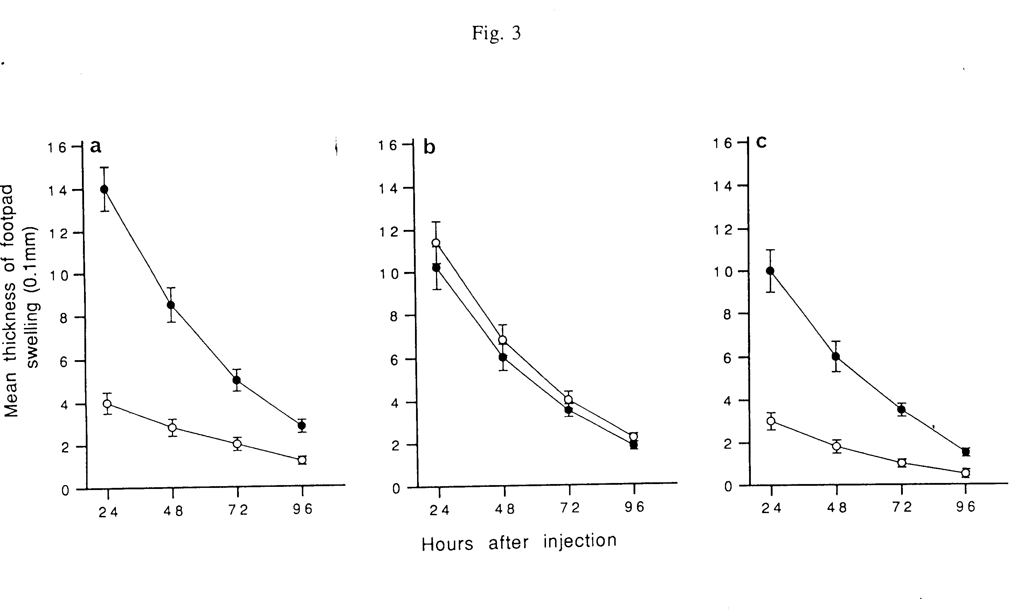

Fig. 3 is a graph showing a footpad response of C₅₇BL/6 female mice to sheep red blood cells (SRBC) and adult *Paragonimus westermani* antigens. The mice were immunized with SRBC one day after an injection of the present cysteine protease. Group treated with the protease on day-1 (○) and control group (●). Bars represent standard errors. (a) The initial SRBC injection one day after the protease treatment. (b) Adult *P. westermani* antigen injection six months after the initial SRBC immunization. (c) SRBC injection one year after the initial SRBC immunization.

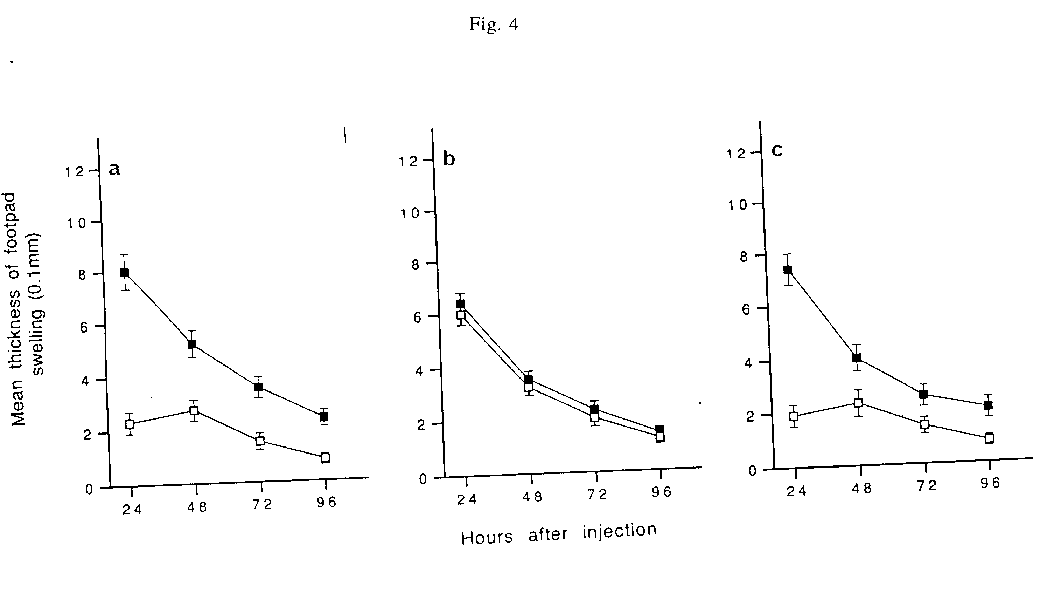

Fig. 4 is a graph showing a footpad response of C₅₇BL/6 male mice to adult *Paragonimus westermani* antigens and sheeps red blood cells (SRBC). The mice were immunized with adult *Paragonimus westermani* antigens one day after injection of the present cysteine protease. Group teated with the protease on day-1 (□) and control group (■). Bars represent standard errors. (a) The initial adult *P.wetermani* antigen injection one day after the protease treatment. (b) SRBC injection six months after the initial adult *P.westermani* antigen immunization. (c) adult *P.westermani* antigen injection one year after the initial adult *P. westermani* antigen immunization.

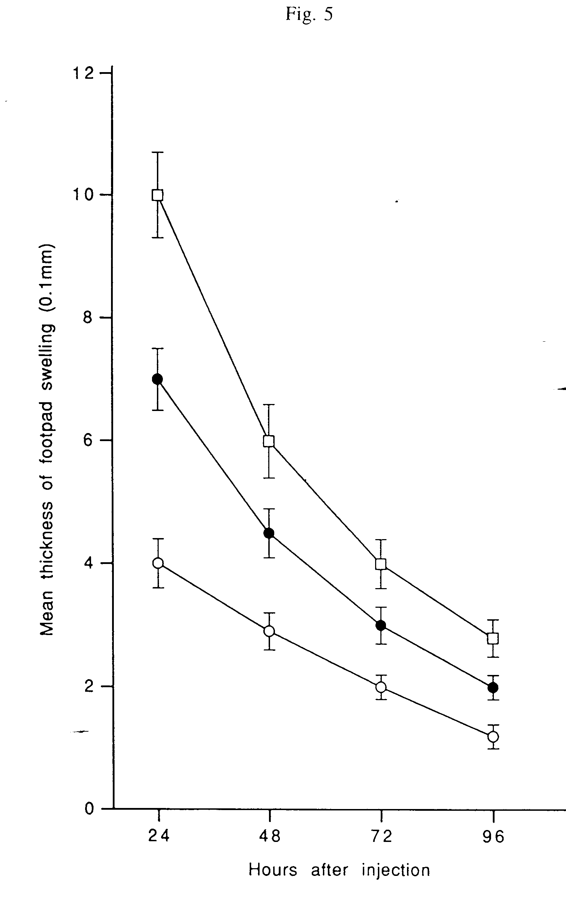

Fig. 5 is a graph showing a footpad response of C₅₇BL female mice to sheep red blood cells (SRBC). The mice were immunized with SRBC one day after injection of various cysteine proteases. Groups treated with plasmin, trypsin, papain, cathepsin B₁ and untreated control (□), collagenase (●), and *P. westermani* cysteine protease (○). Bars represent standard errors.

### Detailed Description of the Invention

It is an object of the present invention to provide immunosuppressive drugs which, without long continuous administration, suppress both delayed-type hypersensitivity and antibody production against specific antigens and graft tissues and induce innunological tolerance to specific antigens and grafted tissues.

The present invention is characterized by the following description.
(1). The present invention provides an immunosuppressive drug in which cysteine protease comprises its composition.
(2). The present invention provides the immunosuppressive drug of (1) in which the cysteine protease contains a larger amount of acidic amino acids than basic amino acids.
(3). The present invention provides the immunosuppressive drug of (1) or (2) in which the cysteine protease is active in neutral hydrogen ion concentration.
(4). The present invention provides the immunosuppressive drug of (1), (2), or (3) in which the cysteine protease is extracted from the infected larvae of parasitic helminths.
(5). The present invention provides the immunosuppressive drug of (1), (2), (3), or (4) in which the cysteine protease comprises an amino acid sequence described in SEQ ID No. 1.
(6). The present invention provides a cysteine protease having an amino acid sequence described in SEQ ID No. 1.
(7). The present invention provides a cysteine protease gene encoding a polypeptide having an amino acid sequence described in SEQ ID No. 1.
(8). The present invention provides a cysteine protease gene having a base sequence described in SEQ ID No. 2.

The term, "cysteine protease" of the present invention is intended to mean an endopeptidase comprising cysteine residues at amino acid positions, 22, 25, 56, 63, 96, 154, and 202, and a histidine residue at amino acid position 161 (the position is numbered from N terminus in the amino acid sequence) and having an active center at cysteine and histidine residues.

The immunosuppressive drug of the invention comprises cysteine protease directly obtained by extracting the cell and tissue of organisms including invertebrates such as parasitic helminths to vertebrates such as various animals including mammal, or by cultured cells isolated from organisms described above, or by genetic engineering using recombinant DNA containing a cysteine protease gene (recombinant DNA technique), or by chemical synthesis.

When the cysteine protease of the present invention (hereinafter referred to as "present cysteine protease") is injected within a few days and/or several days before an injection of an antigen, the injection of the present cysteine protease suppresses cell-mediated immune response and/or humoral immune response, which is antibody production, against the antigen, for a long period of time. In addition, .such a prior injection of the present cysteine protease allow hosts to take a graft for a long period of time and induce the hosts to acquire immunological tolerance to the graft.

Although the detailed mechanism of how cysteine protease works on the immune system is not clearly understood, it is believed that the immunosuppressive drug of the invention administered to a mammalian host is involved with the antigen presentation of immunocytes, receptors or suppressor T cells, thereby suppressing both delayed-type hypersensitivity and antibody production and inducing the immunological tolerance of the mammalian host.

The present invention will be further described in Examples, which are not intended to limit the scope of the invention.

### Example

### 1. Extraction and Purification of Cysteine Protease from Parasitic Helminth

The cysteine protease of the invention was obtained by extracting *Paragonimus westermani* metacercaria with 50 mM imidazole- HCl/pH7.0, subjecting the extract to centrifugation (at 105,000 g, for 60 minutes), loading the supernatant onto Arginine-Sepharose CL-4B affinity chromatography, filtering the eluate on an Ultrogel AcA-54 gel and purifying the filtrate by DEAE-toyopearl S column chromatography. N-terminal 25 amino acids of the purified native cysteine protease is shown in SEQ ID No. 5.

### 2. Amino Acid and Base Sequence of Present Cysteine Protease

The purified present cysteine protease was emulsified with Freund's complete adjuvant. The emulsion was intravenously injected in the ears of rabbits for immunization, and antiserum against the present cysteine protease was obtained.

About 2 X 10⁴ *Paragonimus westermani* metacercariae were used to obtain mRNA. Total RNA (about 100 µg) was extracted from the pooled material by phenol extraction and poly A⁺ RNA (about 2 µg) was recovered by passing the total RNA through an oligo (dT)-cellulose column. The mRNA thus obtained was used to construct a cDNA library using the λgt11 vector. The cDNA library was screened by the antiserum and 25 independent positive clones containing about 300-600bp long insert were obtained. Of 25 clones, two clones having the longest cDNA sequence were designated as λPW6 and λPW24. The two clones were subcloned using pUC 118, and the two subclones were named pPW6 and pPW24, respectively. Then, the base sequences of the two clones were determined by the dideoxy nucleotide sequencing method using a T7 sequencing kit (United States Biochemicals, U.S.A.).

*E. coli* MV1184 was transformed with the subclone pPW6 and the transformant, *E. coli* MV1184/pPW6, was deposited with Fermentation Research Institute, Agency of Industrial Science and Technology and was assigned the accession number FERM BP-3937.

The base sequence and deduced amino acid sequence of pPW6 and pPW24 are shown in SEQ ID Nos. 3 and 4, respectively. Primers were synthesized based on the sequence near the ends of the cDNA insert of the clone and were used to screen the cDNA library again by the PCR.

A base sequence including the entire coding region of the gene of one mature cysteine protease of *Paragonimus westermani* metacercaria was determined (SEQ ID No. 2). The mRNA sequence contains an open reading frame with a significant length. The deduced amino acid sequence is shown in SEQ ID No. 1. The base sequence of the other several cDNA clones strongly suggested that each protease has a few different amino acids and belongs to a closely related protein family. The mature cysteine protease of the invention was found to contain 215 amino acids.

### 3. Characterization of Amino Acid Sequence of Present Cysteine Protease

The comparison of the primary structures of the present cysteine protease and the cysteine protease of other species (hereinafter referred to as "similar cysteine proteases") has revealed the following features: an amino acid sequence (boxed region) in Fig. 1 is homologous in both the present cysteine protease and similar cysteine proteases; cysteine residues are found at amino acid positions, 22, 25, 56, 63, 96, 154, and 202, and a histidine residue is found at amino acid position 161, the position that is numbered from N terminus in the amino acid sequence. This primary structure suggests that the present cysteine protease has a typical feature of cysteine protease. However, the underlined amino acid sequence of the present cysteine protease is different from those of all the other similar cysteine proteases.

In addition, the base sequences of other clones have revealed some substitutions of amino acid residues: in the present cysteine protease, there are substitutions, Ala→ Pro (amino acid position 15), Ser→ Glu (21), Arg→ Met (58), Val→ Ala (59), Gln→ Glu (61), Ala→ Ser (69), and Tyr→ Asp (77), the position that is numbered from N terminus in the amino acid sequence. This amino acid substitution strongly suggests that the primary structure of the present cysteine protease is at least 90% homologous to the other cysteine protease and that the present cysteine protease has a few different amino acids from other cysteine proteases to form its own family. These findings also coincide with variability found in the serine proteases of nematodes and are believed to be the common feature of parasitic helminths.

Furthermore, there is a difference in the number of amino acid residues between cysteine at amino acid position 56 and the following cysteine: 6 amino acid residues are found in the present cysteine protease, papain, chymopapain and papaya proteinase while 8 amino acid residues are found in cathepsin L and H. Based on the known three-dementional structure of papain, it is believed that the present cysteine protease forms a disulfide bridge at amino acid positions 22 - 63, 56 - 96 and 154 - 202, and its active site residue is cysteine at amino acid position 25 and histidine at 161.

The present cysteine protease is composed of more acidic amino acids(14.89-14.96 mole % acidic amino acid such as glutamic acid and aspartic acid) than basic amino acid (10.17-10.23 mole % basic amino acid such as lysine and arginine). The amino acid composition is similar to that of cathepsin L, but different from those of papain, chymopapain and papaya proteinase, which contains more basic amino acids (13.66-14.29 mole %) than acidic amino acids (7.08-8.37 mole %) (see Table 1). In order that a cysteine protease works well in the neutral or faint acidic environment of the organism, it appears reasonable that the cysteine protease have a high acidic amino acid content. The high acidic amino acid content of the present cysteine protease is believed to be the reason that the present cysteine protease alone has an immunosuppressive effect while other similar cysteine proteases such as papain and the like do not.

The present cysteine protease contains leucine in a large amount (8.10 - 8.14 mole %) and asparagine and tyrosine in a small amount - 3.86 and 5.95 - 6.58 mole %, respectively), a prominent feature that distinguishes the present cysteine protease from other similar cysteine proteases (Table 1).

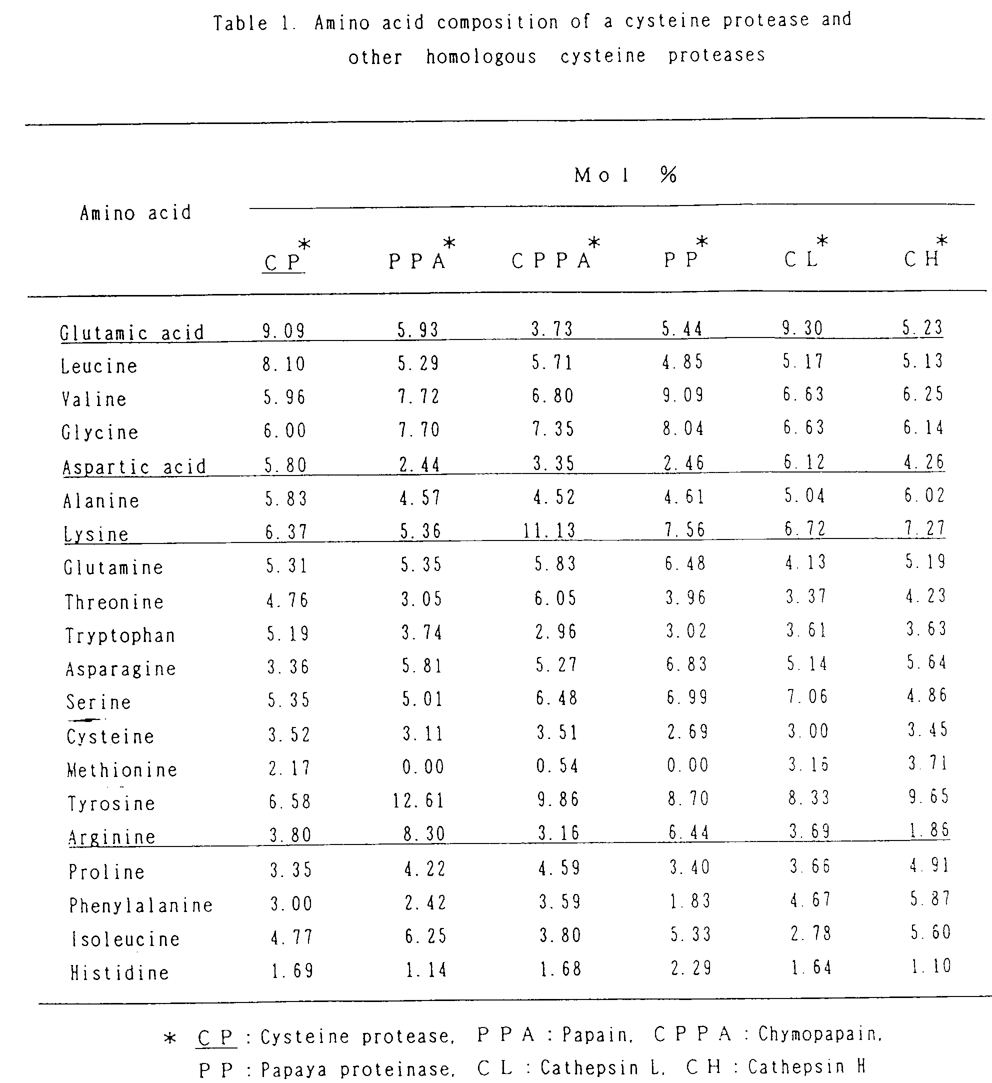

### 4 Suppressive Effects of Present Cysteine Protease on Cell-mediated Immune Response and Humoral Immune Response.

12-week old C57BL/6 female mice were divided into 10 groups, each group having 6 mice. The present cysteine protease was intraperitoneally administered (100 ng protein per mouse which is an enough concentration to suppress footpad reaction) to the mice, except for a control group, 1, 2, 3, 4, 5, 7, and 8 days before the administration of antigens and on the same day when antigens were administered. The mice were immunized with the intraperitoneal injection of 1X10⁸ sheep red blood cells (SRBC) suspended in 0.1 ml of phosphate buffed saline (PBS) as an initial antigen. Five days later, 5X10⁷ SRBCs suspended in 0.05 ml PBS as booster was injected to the footpad. Then, degree of swelling (thickness) was measured (Experimental schedule 1 shown in Table 2).

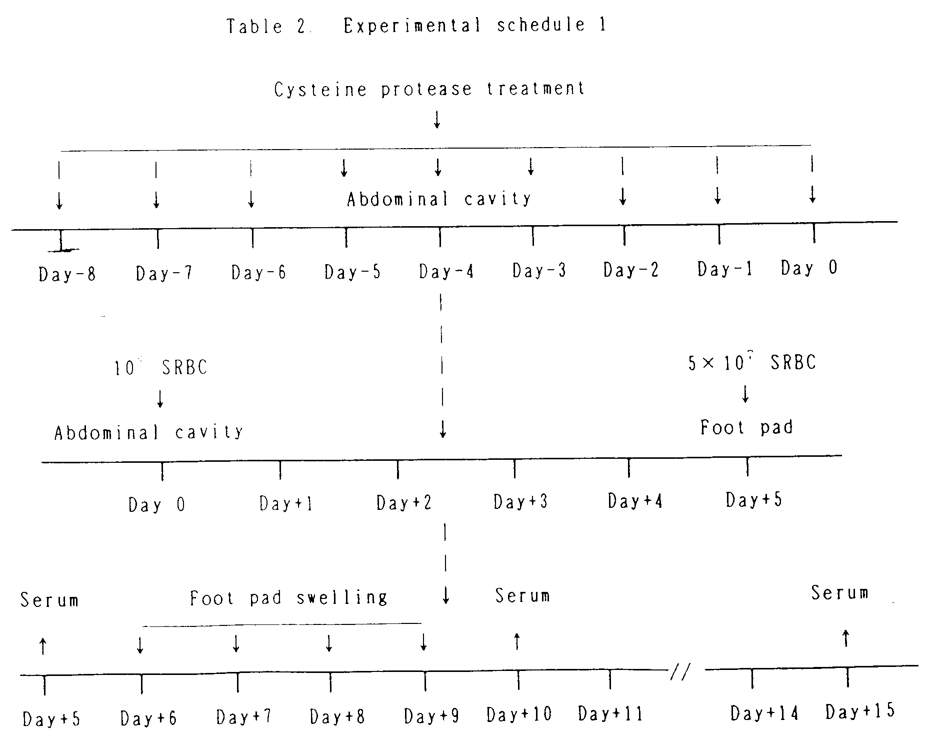

Compared with control, footpad reaction (swelling) was significantly suppressed in the group (P< 0.01) to which present cysteine protease was administered one day before immunization. This result suggests that administration of the present cysteine protease suppresses delayed-type hypersensitivity, against SRBC (Fig.2).

Each group of mice was tested for the antibody titer (HA) of blood serum using SRBC. Compared with control, antibody production was significantly suppressed in the group of mice to which the present cysteine protease was administered 4 - 5 days before the injection of the antigen (Table 3).

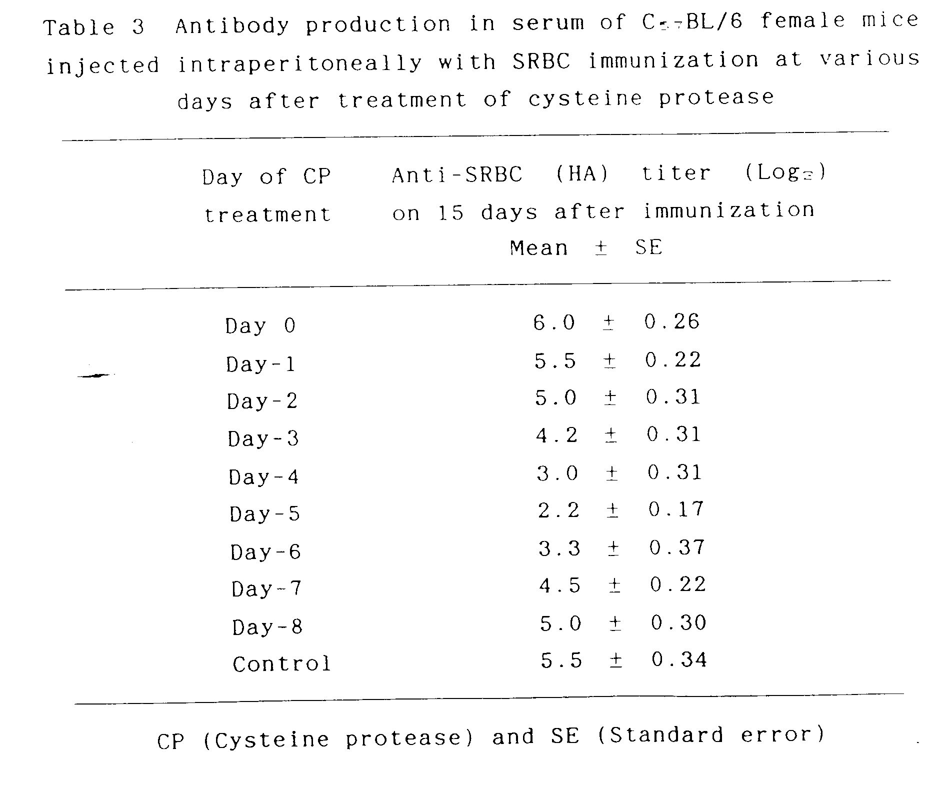

### 5 Immunological Tolerance Induced by Present Cysteine Protease

The present protease (100 ng protein per mouse) was intraperitoneally administered to 12-week old B57BL/6 female and male mice. One day later, a group was immunized with SRBC or adult *Paragonimus westermani* antigens (Experimental schedule 2 shown in Table 4). The group demonstrated the suppression of a footpad reaction against SRBC or adult *Paragonimus westermani* antigens even one year after the initial immunization (Figs 3 and 4). In these groups, the immunosuppression was specifically occurred to the antigen that was initially injected before one year while no immunosuppression of a footpad reaction was occurred to other antigens (Figs.3 and 4).

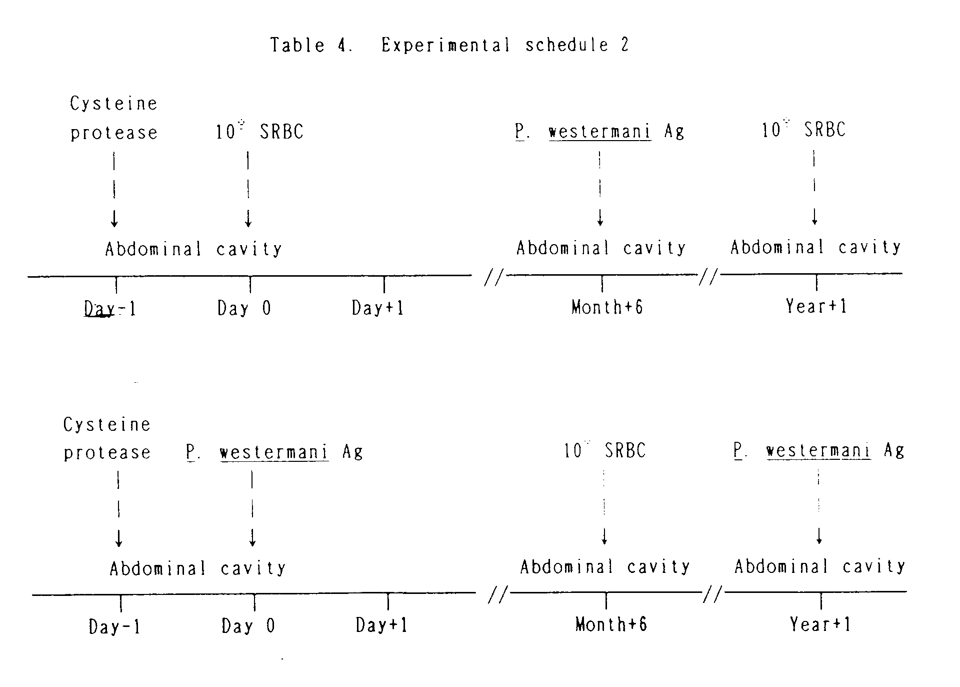

As is shown in Experimental schedule 3 in Table 5, when the skin of 8-week old, AKR female mice was implanted to 10-week old, C3H/He female mice to which the present cysteine protease (total amount, 1.5 µg protein) had been administered one and 4 days before implantation, the administered group took the graft for a significantly longer period of time (P< 0.05-0.01; mean survival time: 100±36) than control (mean survival time: 18±0.5 days) (Table 6).

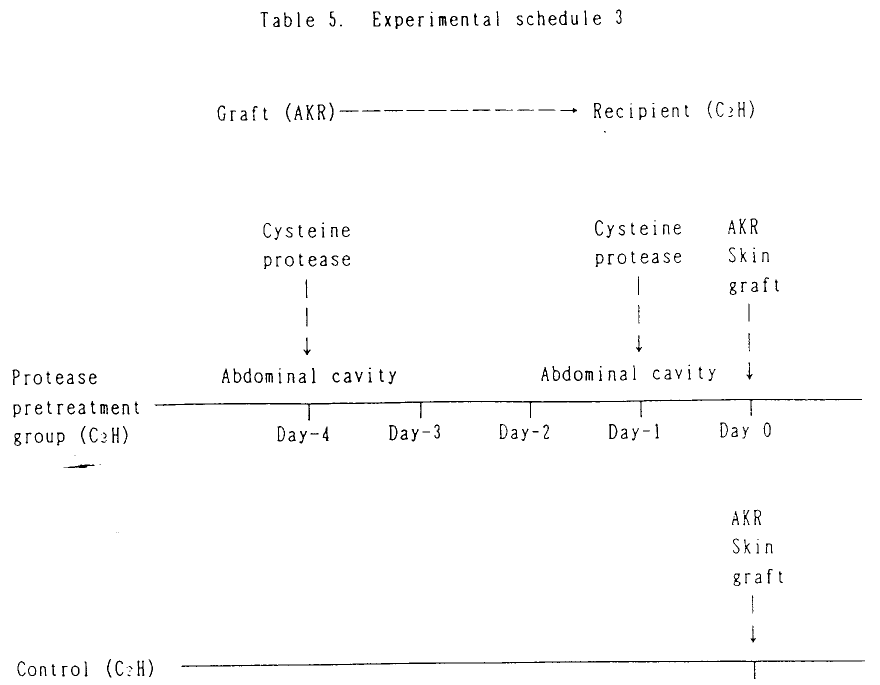

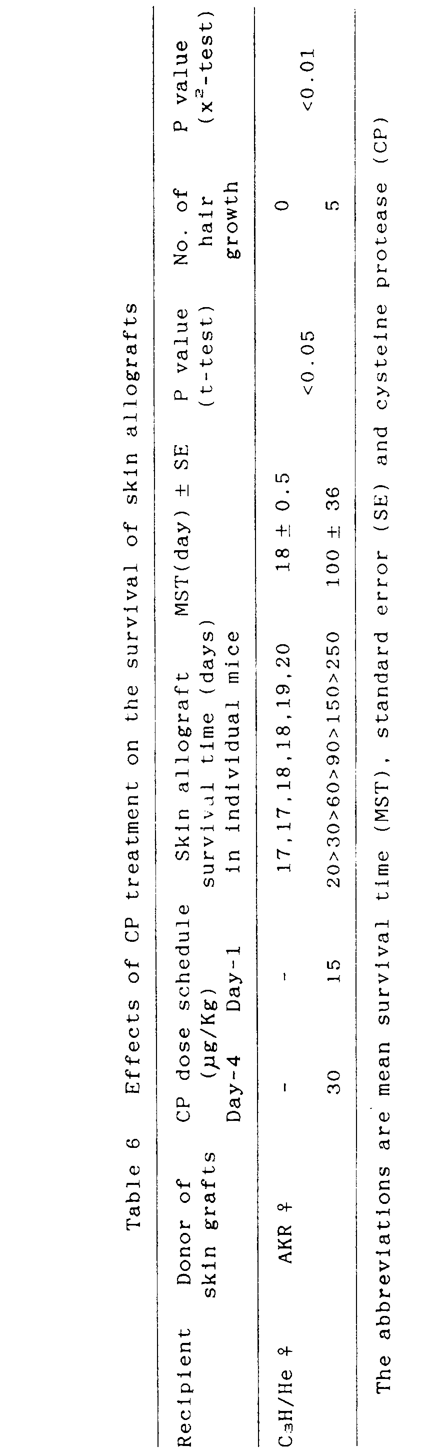

As is evident from the results, the present cysteine protease intraperitoneally administered to mice was found to induce immunological tolerance to the antigen or the tissue which were injected or implanted immediately after the administration of the present cysteine protease.

### 6. Immunological Tolerance of Other Protease Species

Most of the different proteases (e.g., 100 ng of Plasmin, Trypsin, Cathepsin B1, Papain, or Collagenase per mouse) intraperitoneally administered to 12-week old, C57BL/6 female mice did not induce immunological tolerance (Fig. 5). Weak suppression of a footpad reaction was observed in the collagenase administered group but no immunosuppression was observed one year later.

As is evident from the above experiment, the administration of the present cysteine protease suppresses delayed-type hypersensitivity and antibody production against specific antigens and implants, and induces immunological tolerance in the mice.

Pharmaceutical agents utilizing the immunosuppressive effects of the present cysteine protease for therapeutic purpose may be in various forms including an injectable solution described in Example, inhalant, ointment, or a lyophilized form. Dosage may depend on how the present cysteine protease is administered and what therapeutic purpose one is in need.

Although the present cysteine protease used in Example is one that is directly extracted from parasitic helminths, an amount of the cysteine protease that is extracted from one parasitic helminth is a very small. For the isolation and purification of the cysteine protease, it not only takes long time but is also costly so that it is desirable for a commercial scale of the preparation of the cysteine protease to produce it in a large amount by cultured animal cells or by genetic engineering using recombinant DNA containing a gene of interest (recombinant DNA technology).

## Claims

1. A cysteine protease for use in treatment of the human or animal body by therapy.

2. A protease according to claim 1 for use in suppressing the human or animal immune system.

3. A protease according to claim 1 or 2 which is capable of inhibiting footpad swelling or antibody production in an animal to which an antigen has been administered.

4. A protease according to claim 1, 2 or 3 which is active at neutral pH.

5. A protease according to any one of the preceding claims obtainable from the larvae of parasitic helminths.

6. A cysteine protease having the sequence depicted in SEQ ID No. 1.

7. A pharmaceutical composition comprising a pharmaceutically acceptable carrier or diluent and, as active ingredient, a protease as claimed in any one of the preceding claims.

8. A recombinant DNA molecule encoding a protease as claimed in any one of claims 1 to 6.

9. A recombinant expression vector encoding a protease as claimed in any one of claims 1 to 6.

10. A DNA molecule according to claim 8 or a vector according to claim 9 comprising the sequence depicted in SEQ ID No. 2.

11. A host cell transformed or transfected with a vector as claimed in claim 9 or 10, and which is capable of producing a protease as claimed in any one of claims 1 to 6.

12. Use of a protease as claimed in any one of claims 1 to 6 for the manufacture of a medicament for suppressing the human or animal immune system.

13. A cysteine protease capable of suppressing the human or animal immune system.
